# EUROPEAN PATENT APPLICATION

(11) **EP 4 374 853 A1**
(43) Date of publication of application: **29.05.2024**
(21) Application number: 22208932.8
(22) Date of filing: 22.11.2022
(51) Int. Cl.: A61K 9/20

(54) **SOLID FORMULATION OF ENZALUTAMIDE**

(71) Applicant: Lotus Pharmaceutical Co., Ltd., 11046 Taipei City (TW)
(72) Inventor: Wang, Chris, Tuku Township (TW); Gattani, Yogesh Sevaramji, Nantou City (TW); Gupta, Vijender, Nantou City (TW); Chawla, Manish, Nantou City (TW)
(74) Representative: Harber IP s.r.o.

(57) **Abstract**

The present invention provides a solid pharmaceutical composition comprising a premix containing enzalutamide and hydroxypropylmethyl cellulose phthalate (HPMC-P). The composition may further contain at least one filler, at least one disintegrant, at least one glidant and/or at least one lubricant. The composition has similar dissolution properties to the reference product, while simultaneously showing a very good bioavailability.

## Description

### Technical Field

The present invention relates to a new composition of enzalutamide which obviates the poor solubility of enzalutamide by preparing premix with polymers. A significant advantage of the composition of the invention is that it enables to modulate enzalutamide dissolution rate.

### Background Art

Enzalutamide, chemical name 4-{3-[4-cyano-3-(trifluoromethyl)phenyl]-5,5-dimethyl-4-oxo-2-sulfanylideneimiidazolidin-1-yl}-2-fluoro-N-methylbenzymide, is a white to off-white, non-hygroscopic crystalline solid. It is practically insoluble in aqueous media between pH 1-11 but has high permeability and is classified as BCS class II drug. Thus, the major problem of the active pharmaceutical substance are its dissolution characteristics rather than its absorption.

Currently, enzalutamide is sold under the brand name Xtandi. Xtandi is indicated for the treatment of adult men with high-risk non-metastatic castration-resistant prostate cancer (CRPC); the treatment of adult men with metastatic CRPC who are asymptomatic or mildly symptomatic after failure of androgen deprivation therapy in whom chemotherapy is not yet clinically indicated; and the treatment of adult men with metastatic CRPC whose disease has progressed on or after docetaxel therapy. WO 2006124118 claims enzalutamide (RD162'), salts thereof, and its use in the treatment of prostate cancer. Recommended daily dose is 160 mg enzalutamide. A composition with best bioavailability is in the form of enzalutamide solution in soft gelatine capsules. The maximum dose of enzalutamide in one capsule is only 40 mg. Recently, a new formulation i.e. film-coated tablets of strength 40 and 80 mg was made available. Enzalutamide daily dose is four 40 mg soft capsules or film-coated tablets, or two 80 mg film-coated tablets once a day.

Generally, the amorphous solid state offers improved apparent solubility and dissolution rate due to the lower energy barrier required to dissolve molecules and hence transformation of crystalline drug into amorphous is widely employed for increasing solubility. In the polymeric spray drying, drug is incorporated as molecular dispersion in a glass polymeric matrix, stabilized by physical separation of molecules inside the polymer chains. Polymers act as stabilizers by decreasing molecular mobility, and hence inhibit nucleation and crystal growth, while molecular drug-polymer interactions can further inhibit recrystallization.

WO 2014043208 proposes a composition of enzalutamide with an increased solubility. The composition contains an enzalutamide solid dispersion with a polymer. Specifically, the polymer hydroxypropylmethyl cellulose acetate succinate (HPMCAS) is strongly preferred in this document. Only one tested example was close to the solubility of known soft gelatine capsules with Labrasol solution, which was 25% enzalutamide in the solid dispersion in HPMCAS-M. The release behaviour of the solid dispersion of HPMCAS-M is pH dependent. In particular, solid dispersions with Eudragit showed a decreased enzalutamide solubility, and solid dispersions with polyvinylpyrrolidone (PVP VA64) showed an unsatisfactory release profile.

WO 2018199282 discloses an orally administrable pharmaceutical composition containing enzalutamide and polyvinyl alcohol solid dispersion, especially prepared by hot melt extrusion. Unlike the preferred compositions according to the document WO 2014043208, compositions with polyvinyl alcohol show a pH independent release.

WO 2021240206 describes a pharmaceutical composition comprising at least one anionic polymer and/or at least one non-ionic polymer. Particularly preferred is the combination of Eudragit L100-55 and Kollidon VA64

The present invention aims at providing an oral pharmaceutical composition allowing a sufficient drug loading, improved bioavailability, and offering the possibility to modulate enzalutamide dissolution rate.

### Disclosure of the Invention

The present invention focuses on providing a pharmaceutical composition comprising enzalutamide as the active pharmaceutical ingredient (API) with improved bioavailability. This aim is achieved by a formulation comprising an API-polymer premix. The API-polymer premix may be formed by evaporation, fluid bed processing, or spray drying.

In one aspect of the invention, the pharmaceutical composition contains an enzalutamide-polymer premix wherein the polymer is hydroxypropylmethyl cellulose phthalate.

The mass ratio of enzalutamide to the polymer in the premix is from 1:1 to 1:6, preferably from 1 : 3 to 1 : 5.

The premix is formed by evaporation of solvent(s), by fluid bed processing, and/or by spray drying. Spray drying is a preferred method.

In a particular aspect, the present invention relates to a composition comprising enzalutamide as an active ingredient in a premix with a polymer hydroxypropylmethyl cellulose phthalate.

The composition of the invention may further comprise pharmaceutically acceptable excipients. The composition of this particular aspect has an improved bioavailability, and additionally allows to modulate the dissolution and release behavior of the pharmaceutical formulation, in particular by the ratio of enzalutamide and hydroxypropylmethyl cellulose phthalate and by pH.

The term "premix" is understood as a solid phase, in which small particles of the active ingredient are homogenously dispersed within a continuous phase formed by an excipient, in the present invention the excipient is a polymer. Preferably, the particles are at the molecular level; this premix is called "molecular dispersion". A premix is typically prepared by spray drying but may be prepared also by other procedures such as fluid bed processing or evaporation. The premix is in the form of a granulate, and it may be considered to be an intragranular phase.

The composition according to the particular aspect of the invention releases the API pH dependently, the ratio of the API:polymer can modulate the drug releasing curve. This allows the skilled person to tailor the properties of the compositions as needed and as required. The examples of the present invention show several compositions which were designed to achieve more specific dissolution curves.

In a preferred embodiment, the invention also provides mass ratios of the active ingredient enzalutamide , and the polymer hydroxypropylmethyl cellulose phthalate (HPMC-P). The mass ratio of enzalutamide:HPMC-P is preferably from 1 : 2 to 1 : 6, more preferably from 1 : 3 to 1 : 5.

The composition according to the invention typically further contains one or more further pharmaceutically acceptable excipients. Preferred excipients are at least one filler, at least one disintegrant, at least one glidant and/or at least one lubricant. The most preferred further excipients are microcrystalline cellulose, croscarmellose sodium, colloidal silica and magnesium stearate.

The composition is further packed preferably in ALU-ALU blisters.

The composition of the present invention is preferably an oral composition, most preferably a tablet or a coated tablet.

The invention also relates to a method of preparation of the composition according to the invention. The preparation process contains the following steps:
(a) preparation of a premix containing an API and a polymer, preferably by spray drying;
(b) blending the premix with further pharmaceutically acceptable excipients; and
(c) slugging (dry granulation) of the mixture formed in step (b).

These steps may optionally be followed by final blending and compression into tablets. If coated tablets are intended, the compression step is followed by a step of coating the tablet.

The premix in step (a) can be prepared by evaporating a solution of API and the polymer; or spraying the polymer and API solution to the fluid bed; or preferably by spray drying a solution or suspension of the API and the polymer.

Preferably, the API is enzalutamide and the polymer is hydroxypropylmethyl celullose phthalate.

Solvent for the evaporation or fluid drying or spray draying, in which enzalutamide and HPMC-P are soluble, and which exhibits suitable boiling point, is selected. The solvent has to be liquid at room temperature (20 °C) at normal atmospheric pressure. Preferably and in particular for spray drying, the solvent is selected from ketones, alcohols, esters, water and mixtures thereof. For example, acetone or a mixture of acetone and water can be used.

According to a particularly preferred embodiment, enzalutamide tablets or coated tablets are prepared using spray drying technique and using the polymer hydroxypropylmethyl cellulose phthalate (HPMC-P), grade HP-50 and/or HP-55, with molecular weights in the range 20000-200000.

In a particularly preferred embodiment, the pharmaceutical composition comprises 10-15 wt.% of enzalutamide, 40-65 wt.% of hydroxypropylmethyl cellulose phthalate; 10-30 wt.% of microcrystalline cellulose, 7-13 wt.% of croscarmellose sodium, 0.5-2 wt.% colloidal anhydrous silica and 0.5-2 wt.% magnesium stearate, calculated based on the tablet core (i.e., non-coated tablet) weight. Enzalutamide and HPMC-P form the premix; and the remaining excipients are extra granular. The premix is preferably prepared by spray drying. The composition is preferably in the form of a tablet or of a core of a coated tablet.

Hydroxypropylmethyl cellulose phthalate solubility point is around pH 5.0-5.5, thus hydroxypropylmethyl cellulose phthalate is insoluble in gastric fluid but swells and dissolves rapidly in the upper intestine. Molecular weight of hydroxypropylmethyl cellulose phthalate 20000-200000 is particularly suitable to achieve an excellent amorphization by spray drying. Hydroxypropylmethyl cellulose phthalate has a high Tg (137°C for HP-50 and 133°C for HP-55), and thus their use results in a high Tg of the amorphized drug formulation which prevents re-crystallization.

### Brief description of figures

Fig. 1. Dissolution profile of the inventive composition - 80 mg Enzalutamide tablets containing different types of HPMC
Fig. 2. Dissolution profile of the inventive composition - 160 mg Enzalutamide tablets containing different types of HPMC
Fig. 3. The final dosage form manufacturing process flow diagram.

### Examples

### Reference example 1

Example 6 from WO 2021240206A, comparison with the original (reference) product.

Process of preparation: All starting materials were weighed according to Reference Table 1 below. API (enzalutamide) and polymer according to Table 1 were dissolved in acetone, and spray dried to form a premix. The spray dried premix was blended with other excipients, followed by final blending, compression, and coating.

### Reference Table 1

| | Batch number: | 19006Y1 | 0016P1 (T1) | | | 0017P1 (T2) | | |
|---|---|---|---|---|---|---|---|---|
| | | RLD Xtandi 80 mg | (API:Kollidon VA64: Eudragit L100-55 = 1:1.5:1.5) | | | (API:Kollidon VA64: Eudragit L100-55 = 1:1.875:1.125) | | |
| S. No. | Ingredients | Qty per unit (mg) | Qty per unit (mg) | | Qty per unit (%) | Qty per unit Qty per (mg) unit (%) | | |
| 1 | Enzalutamide | √ | 80 | | 12.3 | 80 | | 12.3 |
| 2 | Kollidon^{®} VA64 | X | 120 | | 18.5 | 150 | | 23.1 |
| 3 | Hypromellose Acetate succinate | √ | X | | X | X | | X |
| 4 | Eudragit L100-55 | X | 120 | | 18.5 | 90 | | 13.8 |
| 5 | Microcrystalline Cellulose | √ | 252 | | 38.8 | 252 | | 38.8 |
| 6 | Croscarmellose sodium | √ | 65 | | 10.0 | 65 | | 10.0 |
| 7 | Colloidal anhydrous silica | √ | 6.5 | | 1.0 | 6.5 | | 1.0 |
| 8 | Magnesium stearate | √ | 6.5 | | 1.0 | 6.5 | | 1.0 |
| | Tablet core weight | - | 650 | | 100.0 | 650 | | 100.0 |
| 9 | Opadry Yellow /Coating material (Coating material composition: Hypromellose Talc, Macrogol (8000), Titanium dioxide, Iron oxide yellow) | √ | 20 | | 3.08 | 20 | | 3.08 |
| | Coated tablet weight | | 670 | | | 670 | | |
| | | | 90% Confidence Interval | | T/R Ratio | 90% Confidence Interval | | T/R Ratio |
| | | | Lower | Upper | (%) | Lower | Upper | (%) |
| | | | (%) | (%) | | (%) | (%) | |
| | | Cmax | 45.54 | 66.72 | 55.12 | 53.71 | 80.12 | 65.6 |
| | | AUCT | 65.42 | 88.85 | 76.24 | 71.95 | 100.81 | 85.17 |

The spray dried material had a very low density and poor flow. Poor flow leads to weight variation during the compression stage.

### Dissolution profile:

The dissolution test was performed in 0.1N HCl with 0.3% CTAB for the first 60 minutes, then changed to pH 6.8 buffer with 0.3% CTAB for the next 30 minutes. The formulations showed similar dissolution profile in comparison with the reference product Xtandi 80 mg (see fig. 12 of WO2021240206A), however, in bioequivalence studies the reference compositions failed.

The Bioequivalence study was conducted based on the European Medicines Agency GUIDELINE ON THE INVESTIGATION OF BIOEQUIVALENCE issued on 20. Jan 2010.

Bioequivalence (BE) study results:
T1 Cmax is 45% Lower than reference product, AUC 24 % lower than reference product
T2 Cmax is 34% Lower than reference product, AUC 15 % lower than reference product

### Example 1: Compositions with spray dried enzalutamide / HPMC-P premix

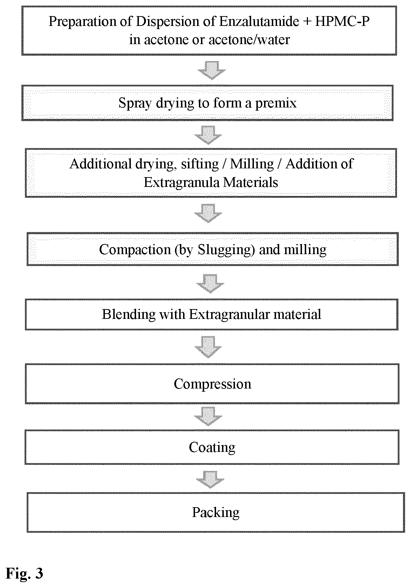

Process of preparation: All starting materials were weighed according to Table 1a. API (enzylutamide) and polymer were dispersed in acetone/water, and spray dried to form a premix. The spray dried premix was blended with extra-granular excipients, followed by slugging (dry granulation), final blending, compression, and coating. The process workflow is shown in Fig. 3.

**Table 1a: Tested compositions**

| Batch number Example | LP111-149 1.1 | LP111-153 1.2 | LP111-154 1.3 | LP111-156 1.5 | LP111-157 1.6 | LP111-163 1.7 | LP111-164 1.8 |
|---|---|---|---|---|---|---|---|
| Formulation Details | API: HPMC-P HP-55=1:3 | API: HPMC-P HP-55=1:4 | API: HPMC-P HP-55=1:2.4 | API: HPMC-P HP-50=1:3 | API: HPMC-P HP-50=1:4 | API: HPMC-P HP-55=1:5 | API: HPMC-P HP-50=1:5 |
| Enzalutamide | 80 | 80 | 80 | 80 | 80 | 80 | 80 |
| HPMC-P HP-50 | - | - | - | 240 | 320 | - | 400 |
| HPMC-P HP-55 | 240 | 320 | 192 | - | - | 400 | - |
| Acetone / Acetone:Water (10 % of solid) | Acetone | Acetone | Acetone | 95:5 | 95:5 | 95:5 | 95:5 |
| Microcrystalline cellulose PH102 | 252 | 172 | 300 | 252 | 172 | 92 | 92 |
| Croscarmellose sodium | 65 | 65 | 65 | 65 | 65 | 65 | 65 |
| Colloidal anhydrous silica | 6.5 | 6.5 | 6.5 | 6.5 | 6.5 | 6.5 | 6.5 |
| Magnesium stearate | 6.5 | 6.5 | 6.5 | 6.5 | 6.5 | 6.5 | 6.5 |
| **Tablet core weight** | 650 | 650 | 650 | 650 | 650 | 650 | 650 |
| Opadry yellow 03F620118 | 3.0% | 3.0% | 3.0% | 3.0% | 3.0% | 3.0% | 3.0% |
| **Coated tablet weight** | 669.5 | 669.5 | 669.5 | 669.5 | 669.5 | 669.5 | 669.5 |

**Table 1b: Spray Dried Premix Properties**

| Batch number Example | | LP111-154 1.5 | LP111-163 1.7 | LP111-164 1.8 |
|---|---|---|---|---|
| | | API: HPMC-P HP-55=1:2.4 | API: HPMC-P HP-55=1:5 | API: HPMC-P HP-50=1:5 |
| Spray Dried material | Bulk density (g/mL) | 0.2214 | 0.1258 | 0.126 |

**Table 1c: Tablet core properties**

| Batch number Example | | LP111-149 1.1 | LP111-153 1.2 | LP111-154 1.3 | LP111-156 1.5 | LP111-157 1.6 | LP111-163 1.7 | LP111-164 1.8 |
|---|---|---|---|---|---|---|---|---|
| Tablet Cores | Weight (mg) | 625-675 | 620-680 | 650-670 | 650-680 | 645-680 | 664 | 650 |
| | Disintegratio n time | 40" - 42" | 40"-50" | 43" | 6' 30"- 1'03"-1'33" | 30"-48" | 49"-58" | 1'02" - 1'10' |
| | Hardness (Kp) | 13-16 | 9.0-13.0 | 12.0-14.0 | 13.0-16.0 | 12.0-15.0 | 15 | 14.3-16.5 |
| | Thickness (mm) | NP | NP | NP | NP | NP | 7.2 | 7.27-7.32 |

| | | | | | | | | |
|---|---|---|---|---|---|---|---|---|
| NP: Not performed DT: disintegration time | | | | | | | | |

**Table Id: Process Parameters for Spray drying**

| Batch number Example | | LP111-149 1.1 | LP111-153 1.2 | LP111-154 1.3 | LP111-156 1.5 | LP111-157 1.6 | LP111-163 1.7 | LP111-164 1.8 |
|---|---|---|---|---|---|---|---|---|
| Process Parameter for Spray drying | Blower (fan) speed Hz | 40 | 40 | 40 | 40 | 40 | 40 | 40 |
| | Inlet Temperature (°C) | 99.7-100.6 | 99.3-105.3 | 99.7-100.8 | 99.4-100.7 | 99.6-100.8 | 100 | 100-110 |
| | Exhaust Temperature (°C) | 43.7-61.8 | 40.9-65.9 | 31.0-44.7 | 54.6-78.2 | 28.5-47.3 | 52-77 | 54-74 |
| | Peristaltic pump speed (rpm) | 10 | 10-15 | 12 | 12 | 12 | 12 | 12 |
| | Deblocker frequency (seconds) | 4 | 4 | 4 | 4 | 4 | 4 | 4 |
| | Spray pressure (MPa) | 0.2 | 0.2 | 0.2 | 0.2 | 0.2 | 0.2 | 0.2 |
| | Oxygen conc. (% Vol)/ only for reference | 0.08-1.97 | 0.35-1.42 | 0.62-1.99 | 0.64-0.92 | 0.59-0.97 | 0.57-0.87 | 0.44-0.83 |

In preliminary experiments, it was observed that when slugging step was not used, the flowability of the final blend was not satisfactory. We surprisingly discovered that flowability of the final blend could be improved by using slugging process (dry granulation) before compression.

DT (disintegration time) of tablets containing HPMC AS (polymer used in RLD) and HPMC-P (polymer used in the developed product) is similar, approximately 1 min.

### Example 2: Compositions comprising spray dried enzalutamide/HPMC-P premix

### Manufacturing process:

Raw materials for premix (enzalutamide, HPMC-P) were weighed according to Table 2, dispersed in mixture of acetone and water and stirred at 200-1500 rpm for 1-2 hours at laboratory temperature. The dispersion was spray dried under the same spray drying conditions as in Example 1, Table 1d, LP111-164. The spray dried premix was sieved through #20 mesh.

Extra granular excipients (except lubricant) were weighed according to Table 2 and sieved through #30 mesh. The excipients were loaded together with the spray dried premix into a blender and mixed at 10-30 rpm for 20-30 mins, followed by slugging (Dry granulation) and milling. Milled material with Magnesium stearate was transferred into the blender and the mixture was mixed at 10-30 rpm for 20-30 mins.

The final mixture was then compressed into tablets.

Coating solution was prepared by adding Opadry Yellow 03F620118 into purified water under stirring for at least 1 hour. Core tablets were transferred into a coating pan, prewarmed and coated up to a weight gain of about 3.08% w/w using the coating solution.

The coated tablets were packed in PVC/Aclar and Alu/Alu blister pack.

**Table 2: Preferred compositions**

| **Sr. No** | **Ingredients** | **Formula 1 Example 2.1 (mg/tab)/ % w/w** | **Formula 2 Example 2.2 (mg/tab)/ % w/w** | **Function** |
|---|---|---|---|---|
| 1 | Enzalutamide (Form A) | 80.00/ 12.31 % | 80.00 | API |
| 2 | HPMC-P HP-55 | 400.00 / 61.54 % | - | Polymer |
| 3 | HPMC-P HP-50 | - | 400.00/ 61.54 % | Polymer |
| 4 | Acetone/water | q.s. | q.s. | Solvent |
| | **Premix Weight** | **480.00** | **480.00** | |
| 5 | Microcrystalline cellulose PH102 | 92.00/ 14.15 % | 92.0 / 14.15 % | Diluent/Filler |
| 6 | Croscarmellose sodium | 65.0 / 10 % | 65.0 / 10 % | Disintegrant |
| 7 | Colloidal anhydrous silica | 6.50 / 1 % | 6.50 / 1 % | Glidant |
| 8 | Magnesium stearate | 6.50 / 1 % | 6.50 / 1 % | Lubricant |
| **Tablet Core Weight** | | **650.00** | **650.00** | - |
| 9 | Opadry Yellow 03F620118 | 20.0 | 20.0 | Coating material |
| 10 | Water^{∗} | q.s. | q.s. | Solvent for coating |
| **Coated Tablet Weight** | | 670.0 | 670.0 | - |

| | | | | |
|---|---|---|---|---|
| ^{∗} Purified water will be evaporated during the drying process; quantity of distilled water is not included in quantity of dosage unit | | | | |

### Dissolution in release condition:

| **Time Example** | **RLD- 20010Y1 RLD** | **C31337P1 2.1** | **C31338P1 2.2** |
|---|---|---|---|
| **Composition** | **Xtandi ^{®} API: HPMC AS** | **API: HPMC-P HP-55** = **1:5** | **API: HPMC-P HP-50** = **1:5** |
| **Condition:** pH 6.8 Phosphate buffer + 0.3% CTAB, 900mL, 75 rpm, USP II with JP sinker | | | |
| | **Avg** | **Avg** | **Avg** |
| 0 | 0 | 0 | 0 |
| 15 | 70 | 80 | 79 |
| 30 | 85 | 86 | 82 |
| 45 | 89 | 87 | 84 |
| | | | |
| 60 | 91 | 89 | 85 |

Bioequivalence study was conducted based on the European Medicines Agency GUIDELINE ON THE INVESTIGATION OF BIOEQUIVALENCE issued on 20. Jan 2010.

### Bioequivalence Study results:

A Pilot Bioequivalence study "An open-label, randomized, balanced, four-treatment, single-period, parallel, pilot, oral bioequivalence study of Enzalutamide film-coated tablets 80 mg and Xtandi^{®} Tablets 80 mg (Enzalutamide) in healthy, adult, human male subjects under fasting conditions"

| **Batch no. Example** | **C31337 2.1** | **C31338 2.2** |
|---|---|---|
| API/ polymer ratio | API: HPMC-P P-55 = 1:5 | API: HPMC-P P-50 = 1:5 |
| No of Subjects | 14 | 14 |
| Fasting Cmax | 103.70 | 124.05 |
| Fastin AUC₀₋₇₂ | 113.87 | 118.98 |
| Tmax (Median) hrs (RLD) | 2.05 (1.50-4.67) | |
| Tmax (Median) hrs (Test) | 2.34 (0.50-4.67) | 2.00 (0.50-4.35) |

### Example 3: Tablets containing 160 mg of Enzalutamide

All starting materials were weighed according to Table 2. Enzalutamide and HPMC-P were dispersed in acetone/water, and spray dried to form a premix. The spray dried premix was blended with extra-granular excipients, followed by slugging (dry granulation), final blending, compression, and coating.

**Table 3**

| **Sr. No** | **Ingredients** | **Formula 1 Example 3.1 (mg/tab)** / **% w/w** | **Formula** 2 **Example 3.2 (mg/tab)** / **% w/w** | **Formula 3 Example 3.3 (mg/tab)** / **% w/w** | **Function** |
|---|---|---|---|---|---|
| **Intra granular (for spray-dried premix)** | | | | | |
| 1 | Enzalutamide (Form A) | 160.0/ 12.31 % | 160.0/ 12.31 % | 160.0/ 14.16 % | API |
| 2 | HPMC-P HP-55 | 800.0 / 61.54 % | - | 800.0 / 70.80 % | Polymer |
| 3 | HPMC-P HP-50 | - | 800.0 / 61.54 % | - | Polymer |
| 5 | Acetone/water | q.s. | q.s. | q.s. | Solvent |
| - | **Premix Granules Weight** | **960.0** | **960.0** | **960.0** | - |

| **Extra granular (Pre-lubrication mixing)** | | | | | |
|---|---|---|---|---|---|
| 6 | Microcrystalline cellulose PH102 | 184.0/ 14.15 % | 184.0/ 14.15 % | 92.0/ 8.14 % | Diluent/Filler |
| 7 | Croscarmellose sodium | 130.0/ 10% | 130.0/ 10% | 65.0/ 5.75 % | Disintegrant |
| 8 | Colloidal anhydrous silica | 13.0/ 1% | 13.0/ 1% | 6.50/ 0.58 % | Glidant |

| **Extra granular (Lubrication)** | | | | | |
|---|---|---|---|---|---|
| 9 | Magnesium stearate | 13.0/ 1% | 13.0/ 1% | 6.50/ 0.58 % | Lubricant |
| **Tablet Core Weight** | | **1300.0** | **1300.0** | **1130.0** | - |

| **Coating** | | | | | |
|---|---|---|---|---|---|
| 10 | Opadry Yellow 03F620118 | 40.0 | 40.0 | 34.0 | Coating material |
| 11 | Water^{∗} | q.s. | q.s. | q.s. | Solvent for coating |
| **Coated Tablet Weight** | | 1340.0 | 1340.0 | 1164.0 | - |

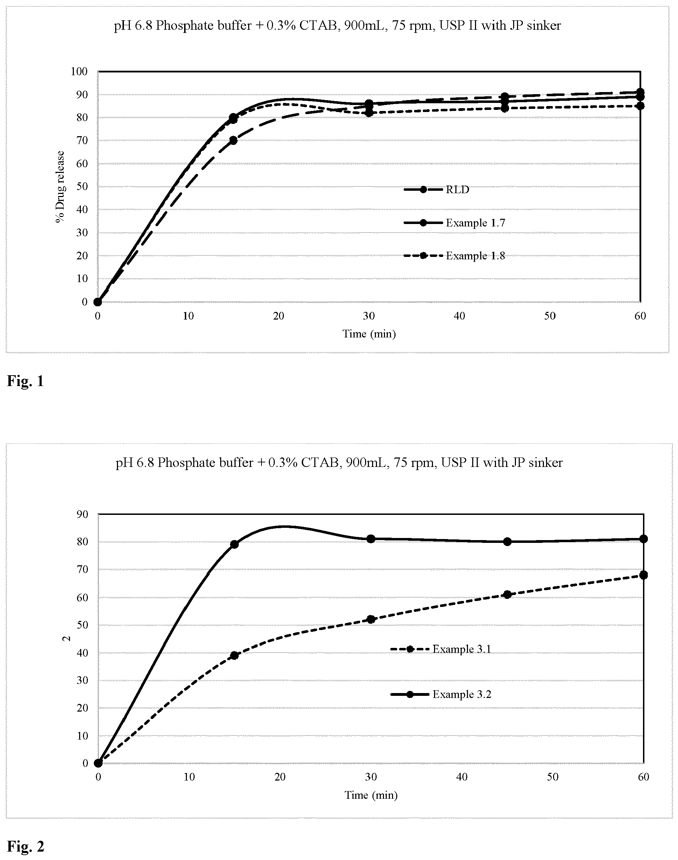

Dissolution profile of the tested formulations in pH 6.8 Phosphate buffer + 0.3% CTAB, 900mL, 75 rpm, USP II with JP sinker is shown on Fig. 2.

## Claims

1. A solid pharmaceutical composition comprising a premix containing enzalutamide and hydroxypropylmethyl cellulose phthalate (HPMC-P).

2. The pharmaceutical composition according to claim 1, wherein hydroxypropylmethyl cellulose phthalate is selected from grades HP-50 and HP-55.

3. The pharmaceutical composition according to claim 1 or 2, wherein the mass ratio of enzalutamide to hydroxypropylmethyl cellulose phthalate is from 1 : 1 to 1 : 6.

4. The pharmaceutical composition according to claim 1 or 2, wherein the mass ratio of enzalutamide to hydroxypropylmethyl cellulose phthalate is from 1 : 3 to 1 : 5.

5. The pharmaceutical composition according to any one of claims 1 to 4, wherein the composition contains one or more further pharmaceutical acceptable excipients selected from at least one filler, at least one disintegrant, at least one glidant and/or at least one lubricant.

6. The pharmaceutical composition according to claim 5, wherein the further excipients are microcrystalline cellulose, croscarmellose sodium, colloidal silica and magnesium stearate.

7. A method for manufacturing of the composition according any one of the previous claims, comprising the following steps:
(a) preparation of a premix containing enzalutamide and hydroxypropylmethyl cellulose phthalate,
(b) blending the premix with further pharmaceutically acceptable excipients; and
(c) slugging of the mixture formed in step (b).

8. The method according to claim 7, further comprising the steps of final blending and compression into tablets.

9. The method according to claim 7 or 8, wherein the premix is prepared by evaporation of solvent(s), by fluid bed processing, and/or by spray drying.

10. The method according to claim 7 or 8, wherein the premix is prepared in step (a) by spray drying of enzalutamide and hydroxypropylmethyl cellulose phthalate.

11. The method according to claim 7 or 8, wherein the premix is prepared in step (a) by spray drying of enzalutamide and hydroxypropylmethyl cellulose phthalate in acetone or acetone/water mixture.
